# EUROPÄISCHE PATENTSCHRIFT

(11) **EP 0 781 112 B1**
(45) Veröffentlichungstag und Bekanntmachung des Hinweises auf die Patenterteilung: **23.12.1998**
(21) Anmeldenummer: 95929748.2
(22) Anmeldetag: 05.09.1995
(51) Int. Cl.: A61B 17/28

(54) **MEDIZINISCHES HANDHABUNGSGERÄT**
MEDICAL MANIPULATOR
MANIPULATEUR MEDICAL

(30) Priorität: 05.09.1994 DE 4431561
(43) Veröffentlichungstag der Anmeldung: 02.07.1997
(73) Patentinhaber: KLINIKUM DER ALBERT-LUDWIGS-UNIVERSITÄT FREIBURG, D-79106 Freiburg (DE)
(72) Erfinder: MATERN, Ulrich, D-79283 Bollschweil (DE); WELLER, Peter, D-81667 München (DE)
(74) Vertreter: Münich, Wilhelm, Dr.
(86) Internationale Anmeldenummer: DE9501196
(87) Internationale Veröffentlichungsnummer: WO9607359

(56) Entgegenhaltungen:
- EP-A- 0 546 767

## Beschreibung

### Technisches Gebiet

Die Erfindung bezieht sich auf ein medizinisches Handhabungsgerät mit einem distal angeordneten medizinischen Instrument einem proximal angeordneten Haltegriff, und einem Schaftteil, das den Haltegriff mit dem Instrument verbindet, gemäß dem Oberbegriff des Patentanspruchs 1.

Derartige Instrumente werden beispielsweise in der Endoskopie und insbesondere in der minimalinvasiven Chirurgie eingesetzt.

### Stand der Technik

Ein medizinisches Handhabungsgerät gemäß dem Oberbegriff des Patentanspruchs 1 ist der EP-A-0 546 767 bekannt. Ein weitgehend ähnliches Handhabungsgerät ist in der DE-A-41 36 861 beschrieben.

Die bekannten Handhabungsgeräte, die insbesondere in der minimalinvasiven Chirurgie eingesetzt werden, haben ein Griffteil in Form eines Pistolengriffs. Das Griffteil ist mit einem Schaftteil verbunden, an dessen distalem Ende ein chirurgisches Instrument angeordnet ist, beispielsweise ein Greifer oder eine Schere. Der Haltegriff weist an der dem chirurgischen Instrument zugewandten Seite des Griffteils einen Betätigungshebel auf, der mit dem chirurgischen Instrument in Übertragungsverbindung steht.

Ungünstig ist bei diesen bekannten Handhabungsgeräten jedoch, daß beim Betätigen des Hebels die Haltestellung der Hand gelöst werden muß, so daß der Haltegriff und das daran fixierte chirurgische Instrument dann nicht mehr sicher gehalten oder geführt werden kann. Um ein Fehlpositionieren des oft nur sehr kleinen chirurgischen Instrumentes und eine damit einhergehende Verletzungsgefahr für den Patienten zu vermeiden, muß deshalb der Haltegriff beim Verstellen oder Auslösen des Betätigungshebels an dem Griffteil oder dem damit verbundenen Schaftteil mit der anderen Hand festgehalten werden. Diese wird jedoch bei minimal invasiven chirurgischen Eingriffen häufig für andere Arbeiten, beispielsweise zum Bedienen von Meß- oder Überwachungsgeräten benötigt.

Der aus der DE-A-41 36 861 bekannte Haltegriff weist ferner an seiner dem chirurgischen Instrument abgewandten Seite zwei mit dem Daumen betätigbare Schalter zum Ansteuern von Elektroantrieben auf, mit denen das chirurgischen Instrument am distalen Schaftende drehbar und verschwenkbar ist. Ungünstig ist dabei jedoch, daß die beiden Schalter mit dem Daumen nur wechselweise betätigbar sind, so daß ein gleichzeitiges Verschwenken und Drehen des chirurgischen Instrumentes praktisch nicht möglich ist. Außerdem muß zum Betätigen der Schalter der Daumen relativ weit von der Hand abgespreizt werden, so daß bei einem minimal invasiven chirurgischen Eingriff, bei dem das chirurgische Instrument mit dem Haltegriff oft länger als eine Stunde vom Chirurg gehalten oder betätigt werden muß, die Gefahr besteht, daß die Hand vorzeitig ermüdet.

### Darstellung der Erfindung

Der Erfindung liegt die Aufgabe zugrunde, ein medizinisches Handhabungsgerät mit einem distal angeordneten medizinischen Instrument einem proximal angeordneten Haltegriff, und einem Schaftteil, das den Haltegriff mit dem Instrument verbindet, anzugeben, mit dem über einen längeren Zeitraum ermüdungsfrei und sicher gearbeitet werden kann. Insbesondere soll das Instrument mit nur einer Hand auf einfache Weise betätigt und/oder positioniert werden können. Außerdem soll außer dem ermüdungsfreien Halten auch ein präzises Positionieren mit dem Handhabungsgerät möglich sein.

Eine erfindungsgemäße Lösung dieser Aufgabe ist im Patentanspruch 1 angegeben. Erfindungsgemäße Weiterbildung dieses Handhabungsgeräts sind Gegenstand der Ansprüche 2 folgende.

Erfindungsgemäß wird ein gattungsgemäßes Handhabungsgerät durch die Kombination folgender Merkmale weitergebildet:
- das Griffteil ist derart ergonomisch geformt, daß es weitgehend mit einer Hand zumindest zwischen Handballen und der Mittelhand bzw. den inneren Fingerhandknochen umgriffen und ohne Einsatz zumindest der äußeren und der mittleren Glieder des Zeigefingers und des Mittelfingers gehalten werden kann,
- zumindest für den Zeige- und den Mittelfinger ist jeweils ein separates Betätigungselement vorgesehen, von denen jedes so ausgebildet ist, daß bei seiner Betätigung mit dem freien Fingerende des zugeordneten Fingers die übrigen Hand- und Fingerbereiche im wesentlichen in Greif- bzw. Haltestellung bleiben.

Das mit den Handballen, der Mittelhand und den inneren Fingerhandknochen umgreifbare, ergonomische geformte Griffteil ermöglicht auch über einen längeren Zeitraum ein ermüdungsfreies, entspanntes Halten des Haltegriffes, wobei die Hand während des Haltens praktisch in ihrer natürlichen Ruhestellung verbleiben kann.

Dabei sind zumindest im Bereich der inneren Fingerhandknochen des Zeige- und Mittelfingers an dem Griffteil Auflageflächen für die Finger vorgesehen, an denen die Finger in Haltestellung bereichsweise anliegen, während im Bereich der freien Fingerenden die Betätigungselemente angeordnet sind. Die Betätigungselemente können dadurch auf einfache Weise bedient werden, ohne daß dazu die Hand aus der Haltestellung gelöst werden muß. In vorteilhafter Weise ermöglicht der Haltegriff dadurch auch während des Betätigungsvorgangs ein sicheres Halten eines an dem Schaftteil vorgesehenen medizinischen Instrumentes. Dabei sind die einzelnen Betätigungselemente jeweils separaten Fingern zugeordnet, so daß mehrere Funktionen, beispielsweise eine Positionierbewegung des medizinischen Instrumentes in mehrereren Achsen (zum Beispiel Drehung und gleichzeitige Neigung) und/oder ein Verstellen des Instrumentes zum Ausführen einer Schneid- oder Greifbewegung, gleichzeitig durchgeführt werden können.

Bei einer vorteilhaften Weiterbildung ist vorgesehen, daß die Betätigungselemente zur proportionalen Übertragung der Betätigungsbewegung ausgebildet sind. Dabei können die Betätigungselemente insbesondere so ausgebildet sein, daß ihre Stellung die Stellung einer diesem Betätigungselement zugeordneten Schwenk- oder Drehachse für das chirurgische oder medizinische Instrument anzeigt.

Diese Ausbildung hat den Vorteil, daß die Bedienungsperson, wie beispielsweise ein Chirurg bei einer minimal invasiven Operation von der Stellung des Betätigungselementes unmittelbar beispielsweise auf die Stellung einer diesem Betätigungselement zugeordneten Schwenk- oder Drehachse für das chirurgische oder medizinische Instrument schließen kann. Damit können Manipulationen auch ohne unmittelbare Sichtkontrolle durchgeführt werden.

Außerdem ermöglicht ein solches analoges Betätigungselement, das beispielweise ein Analogtaster, eine Wippe oder eine analoge Schwenktaste sein kann, ein besonders schnelles Verstellen des medizischen Instrumentes. Bei der Ansteuerung elektrischer Einrichtungen, wie beispielsweise von Motoren oder Aktuatoren und insbesondere distal angeordneten mikromechanischen Aktuatoren können als analoge Stellelemente Drehpotentiometer oder dgl. verwendet werden.

Vorteilhaft ist weiterhin, wenn zumindest für den Zeigefinger und den Mittelfinger, aber auch für den Daumen als Betätigungselemente Tasten, insbesondere Schwenktasten oder Wippen vorgesehen sind, und wenn diese gegen eine Rückstellkraft mit dem jeweiligen Fingerende druckbeaufschlagbar sind. Dabei ist das Gelenk einer solchen Schwenktaste vorzugsweise benachbart zu dem zwischen dem inneren und dem mittleren Fingerhandknochen befindlichen Fingergelenk angeordnet, so daß die Schwenktaste beim Betätigen praktisch die gleiche Schwenkbewegung wie das freie Fingerende ausführt und daher besonders ergonomisch bedienbar ist. Die Schwenktaste kann sich dann über die gesamte Länge der äußeren Fingerhandknochen erstrecken, so daß sich die Druckkräfte beim Betätigen der Schwenktasten auf eine möglichst große Fingerfläche verteilen.

Bei einer weiteren erfindungsgemäßen Weiterbildung ist für den Daumen wenigstens ein separates Betätigungselement, insbesondere eine Drucktaste, eine Schwenktaste oder ein Drehelement vorgesehen. Mit dem Daumen kann dann unabhängig und gegebenenfalls parallel zu dem mit dem Zeige- und Mittelfinger betätigbaren Funktionen eine zusätzliche Funktion, beispielsweise das Verstellen des medizinischen Instrumentes um eine zusätzliche Achse, wie die Längsachse des Schaftteils, ausgeführt werden, ohne daß dazu die Hand aus der Haltestellung gelöst werden muß.

Besonders günstig ist es, wenn im Greifbereich des Daumens mehrere, wahlweise mit dem Daumen verstellbare Betätigungselemente angeordnet sind. So können beispielsweise Schalter zum Ein-/Ausschalten eines HF-Stroms, zum Betätigen einer Absaugung und/oder Spülung sowie Rasten zum Festlegen anderer Betätigungselemente vorgesehen sein, so daß insgesamt eine Vielzahl von Betätigungselementen vorhanden ist, die alle mit nur einer Hand verstellt werden können. Die Elemente, die auch in Art eines sog. Joy-Sticks ausgebildet sein können, können auch mit einem zusätzlichen Schalter, beispielsweise einem Druckknopfschalter ausgestattet sein, mit dem eine Funktion, wie beispielsweise ein Koagulator, ein Laser o. dgl ein- bzw. ausgeschaltet wird.

Eine bevorzugte Weiterbildung der Erfindung sieht vor, daß das Schaftteil zwischen der Auflage bzw. den Betätigungselementen für den Zeigefinger und den Mittelfinger mit dem Griffteil verbunden ist. Das Schaftteil liegt dann in Haltestellung des Haltegriffs etwa in gerader Verlängerung der gestreckten Hand-Unterarmverlaufes. Eine Drehbewegung des Unterarms um dessen Längsachse wird dadurch unmittelbar auf das Schaftteil übertragen, ohne daß dieses dabei seitlich verschwenkt. Das in eine Trokar-Hülse eingesteckte Schaftteil kann dadurch zum Positionieren des medizinischen Instruments auf einfache Weise in der Trokar-Hülse gedreht werden.

Besonders vorteilhaft ist es ferner, wenn das Griffteil mehrteilig ausgebildet ist, und wenn insbesondere mehrere, auswechselbare und unterschiedlich große Ballenteile vorgesehen sind. Der Haltegriff kann dann durch Auswechseln des Ballenteiles auf einfache Weise individuell an die Hand des jeweiligen Benutzers angebracht werden. Der Haltegriff ermöglicht dadurch auch bei Benutzern mit unterschiedlich großen oder unterschiedlich ausgebildeten Händen über einen langen Zeitraum ein ermüdungsfreies Arbeiten.

Eine weitere vorteilhafte Ausführungsform der Erfindung sieht vor, daß zumindest eines der Betätigungselemente mit einer Feststellvorrichtung zum Festlegen des Betätigungselementes in wenigstens einer Endstellung und/oder einer oder mehreren Zwischenstellungen gekoppelt ist. Das Betätigungselement kann dadurch arretiert werden, damit es sich nicht versehentlich verstellt. Außerdem können bestimmte Funktionsstellungen des medizinischen Instrumentes, beispielsweise die Greif- oder Offenstellung eines Greifers, auf einfache Weise fest eingestellt werden.

Besonders vorteilhaft ist, wenn zwischen Daumen und Handwurzelknochen des Zeigefingers ein Klemmsteg mit einem Auflagebereich für den Daumen vorgesehen ist. Der Haltegriff kann dann auch durch Festklemmen des Klemmsteges zwischen Daumen und Handwurzelknochen des Zeigefingers gehalten werden, wobei die Hand vorübergehend aus der Greifstellung gelöst werden kann.

Zweckmäßig ist ferner, wenn im Bereich der Fingerauflagen, insbesondere für den kleinen Finger und den Ringfinger Einformungen bzw. zwischen und/oder neben den Fingern vorstehende Stützstege vorgesehen sind. Der Haltegriff kann dann noch besser gehalten werden, wobei die Stützstege und die Einformungen ein seitliches Abrutschen der Finger verhindern.

Bei einer weiteren erfindungsgemäßen Ausgestaltung ist an dem Griffteil eine Daumenauflage vorgesehen, die vorzugsweise eine Aufnahmevertiefung oder dergleichen Ausformung für den Daumen aufweist. Der Daumen kann dann praktisch in seiner natürlichen Ruhestellung auf der Daumenauflage abgestützt werden, so daß das Griffteil einerseits besonders ermüdungsfrei gehalten werden kann und andererseits die Haltekräfte noch besser auf das Griffteil übertragen werden können.

Besonders günstig ist weiterhin, wenn das medizinische Instrument um die Längsachse des Schaftteiles drehbar an dem Schaftteil gelagert ist, wenn in dem Schaftteil eine Welle vorgesehen ist, die das Schaftteil in Längsrichtung durchsetzt und an ihrem distalen Endbereich drehfest mit dem medizinischen Instrument verbunden ist und wenn die Welle mit einem an dem Griffteil vorgesehenen Betätigungselement in Antriebsverbindung steht. Das medizinische Instrument kann dadurch auf einfache Weise und ohne eine Drehung des Handgriffes oder des Schaftteiles in praktisch beliebige Richtungen in Bezug zur Längsachse des Schaftteiles gedreht werden, wobei der Haltegriff in einer für den Chirurgen günstigen Stellung verbleiben kann. Dabei kann als Betätigungselement zum Verstellen der Richtung des medizinischen Instruments - je nach Anwendungsfall - ein Verstellrad, eine Schwenktaste oder eine mit einem gegebenenfalls in der Drehrichtung umschaltbaren Schrittschaltwerk gekoppelte Drucktaste zweckmäßig sein.

Eine vorteilhafte Ausführungsform sieht vor, daß die Welle direkt oder indirekt über ein Getriebe mit einem vorzugsweise im Daumenbereich an dem Griffteil angeordneten Drehelement oder dergleichen Betätigungselement gekoppelt ist. Das medizinische Instrument kann dadurch um beliebige Drehwinkel um das Schaftteil gedreht werden, wobei das Getriebe vorzugsweise als Übersetzungsgetriebe ausgebildet ist, das den Verstellwinkel des Drehelementes - unter Beibehaltung des Drehsinnes - in einen größeren Verstellwinkel des chirurgischen Instruments übersetzt, damit dieses einfach schnell positioniert werden kann.

Zweckmäßigerweise ist die Welle als Hohlwelle ausgebildet, in deren Innenlängs-Höhlung wenigstens ein Übertragungselement für Zug- und/oder Druckkräfte, insbesondere eine Zug-/Druckstange oder ein Bowdenzug vorgesehen ist. Zusätzlich zu der Drehbewegung können dann weitere Verstellbewegungen auf das medizinische Instrument übertragen werden, die beispielsweise das Öffnen und Schließen eines Greifers und/oder das Verändern des Neigungswinkels eines medizinischen Instruments ermöglichen.

Vorteilhaft ist, wenn in dem Schaftteil wenigstens eine, gegebenenfalls durch die Innenlängs-Höhlung der Hohlwelle gebildete Leitung zum Zu- oder Abführen einer Flüssigkeit - insbesondere einer Spülflüssigkeit - vorgesehen ist. An dem Handgriff können dann entsprechende Betätigungselemente zu der Spülung und/oder Absaugung vorgesehen sein, so daß eine zu behandelnde Operationsstelle unmittelbar vor dem eigentlichen Behandlungsvorgang auf einfache Weise gereinigt werden kann.

Eine Weiterbildung der Erfindung sieht vor, daß an dem Griffteil, vorzugsweise im Bereich des Daumens ein wenigstens in zwei Richtungen seitlich verschwenkbarer Betätigungsknopf angeordnet ist, und daß an dem Betätigungsknopf wenigstens ein mit dem medizinischen Instrument gekoppeltes Übertragungselement angreift. Mit einem solchen Betätigungsknopf können mit nur einem Finger Zug- und Druckkräfte gleichermaßen aufgebracht werden, so daß beispielsweise eine mit dem Betätigungsknopf gekoppelte Schwenkachse für das medizinischen Instrument auf einfache Weise in beiden Richtungen verschwenkbar ist.

Besonders günstig ist, wenn der Betätigungsknopf in allen Seitenrichtungen verschwenkbar ist und mehrere, vorzugsweise vier um jeweils 90 Grad versetzt zueinander an den Betätigungsknopf angreifende Übertragungselemente, vorzugsweise Bowdenzüge aufweist. Der Betätigungsknopf kann mittels der Bowdenzüge und einer beispielsweise an dem distalen Endbereich des Schaftteiles vorgesehenen Schwenkeinrichtung für das medizinische Instrument gekoppelt sein, wobei der jeweilige Schwenkwinkel und die Schwenkrichtung des Betätigungsknopfes in einen dazu korrespondierenden Schwenkwinkel bzw. eine dazu korrespondierende Schwenkrichtung des medizinischen Instrument umgesetzt wird. Der Betätigungsknopf ermöglicht also mit nur einem Finger auf einfache weise eine gleichzeitige Einstellung des Schwenk- und Neigungswinkels des medizinischen Instruments.

Durch die Weiterbildung, bei der das Griffteil zur Reinigung zerlegbar ist und/oder Löcher aufweist bzw. aus Lochstruktur-Elementen besteht, wird die gerade bei medizinischen Instrumenten kritische Reinigung bzw. Sterilisierung erleichtert.

### Kurze Beschreibung der Zeichnung

Nachfolgend sind Ausführungsbeispiele der Erfindung anhand der Zeichnung näher erläutert. Sie zeigen in unterschiedlichen Maßstäben und zum Teil stark schematisiert:
- Fig. 1: eine Seitenansicht des medizinischen Handhabungsgerätes, welche die Innenseiten der Schwenktasten mit Zeige- und Mittelfinger, die Daumenauflage, sowie die Ausformungen für den Ringfinger und den kleinen Finger besonders gut erkennen läßt,
- Fig. 2: eine Aufsicht auf das Handhabungsgerät gemäß Fig. 1, die den an der Oberseite seitlich vorstehenden Klemmsteg und den sich im Bereich seines proximalen Endes in Querrichtung erweiternden Schaftteil besonders gut erkennen läßt,
- Fig. 3: eine Ansicht auf die Innenseite einer Hand mit dem Handhabungsgerät in Haltestellung,
- Fig. 4: die innere Seitenansicht einer Hand mit dem Handhabungsgerät in Haltestellung,
- Fig.5: eine perspektivische Darstellung des Handhabungsgerätes mit demontiertem, strichliniert dargestelltem Ballenteil, wobei die in dem Griffteil enthaltene Mechanik mit eingezeichnet ist und bei der zum Drehen des medizinischen Instruments ein Drehelement vorgesehen ist, und
- Fig. 6: eine Ausführungsform eines Handhabungsgeräts, bei der zum Einstellen des Neigungswinkels und der Neigungsrichtung des medizinischen Instruments ein in allen Seitenrichtungen verschwenkbarer Betätigungsknopf vorgesehen ist.

### Beschreibung von Ausführungsbeispielen

In den folgenden Figuren sind gleiche oder entsprechende Teile jeweils mit den selben Bezugszeichen versehen.

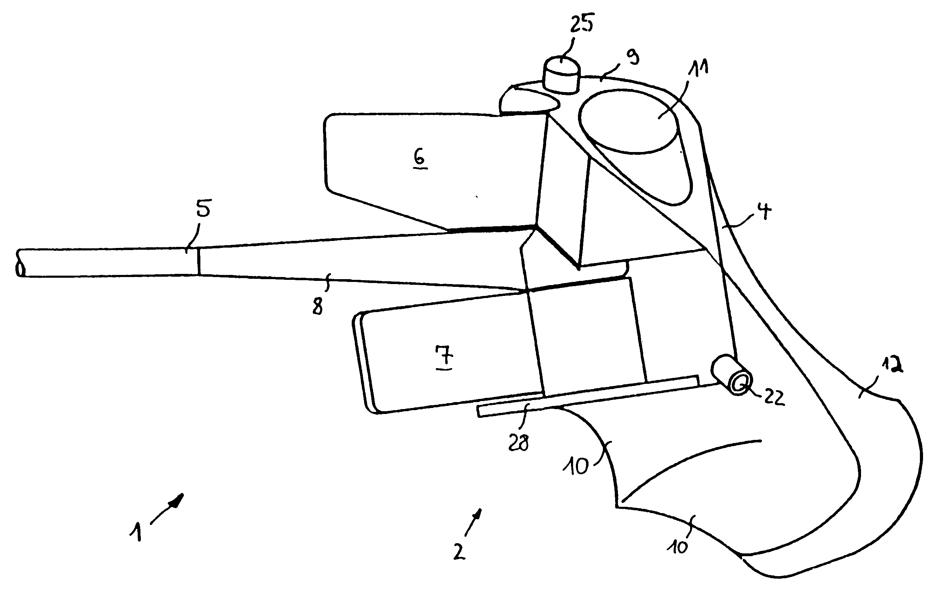

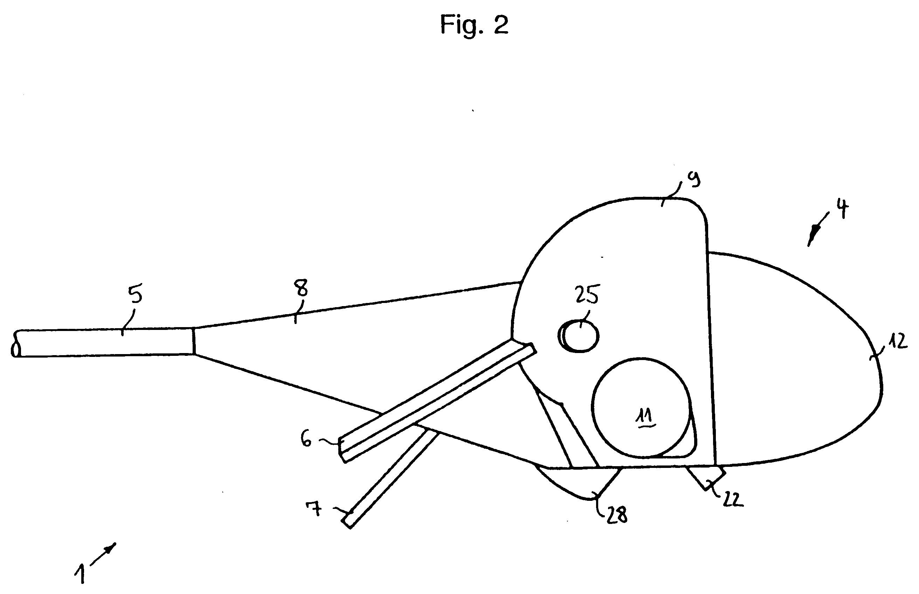

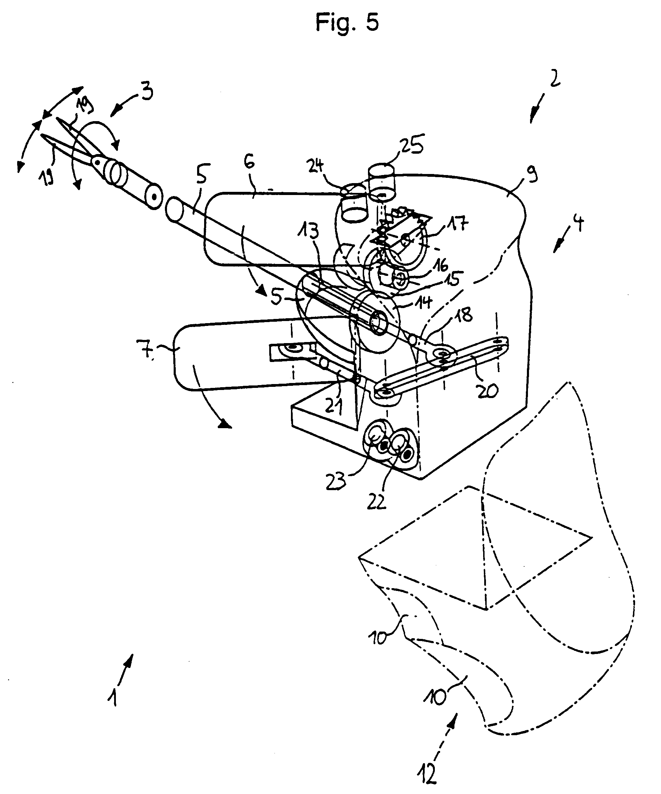

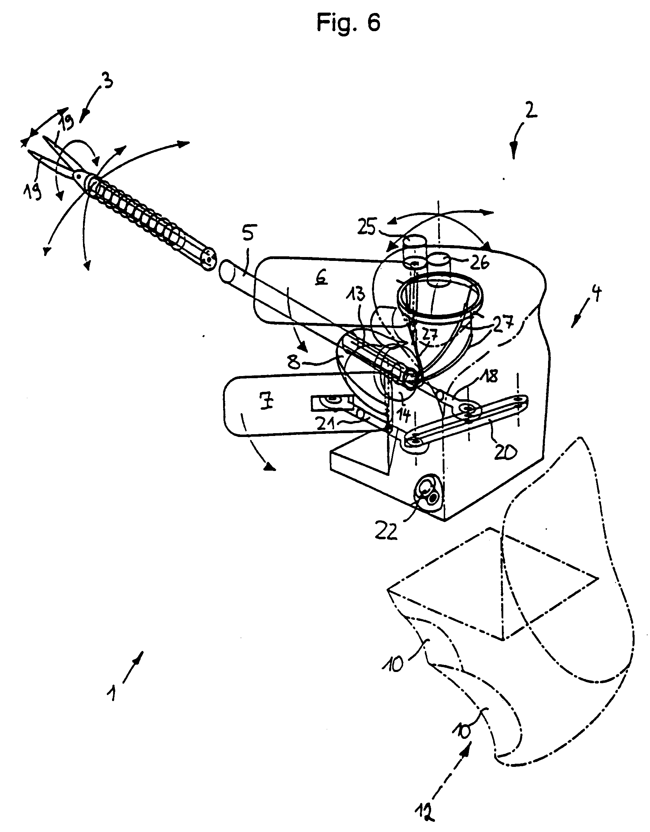

Die Fig. 1 bis 6 zeigen ein im ganzen mit 1 bezeichnetes medizinisches Handhabungsgerät. Das Handhabungsgerät 1 hat einen Haltegriff 2 für ein medizinisches Instrument 3, das lediglich in den Fig. 5 und 6 dargestellt ist. Der Haltegriff 2 weist ein mit der Hand ergreifbares Griffteil 4 auf, an dem mit den Fingern bedienbare Betätigungselemente angebracht sind, die im folgenden noch im einzelnen beschrieben werden

Mit dem Griffteil 4 ist ein Schaftteil 5 verbunden, das an seinem distalen Endbereich das medizinische Instrument 3 trägt. Dabei stehen die Betätigungselemente mit dem medizinischen Instrument 3 in einer Übertragungsverbindung, die beispielsweise mechanisch realisiert ist. Selbstverständlich ist es aber auch möglich, bei distal angeordneten Motoren und/oder Aktuatoren, die beispielsweise mikromechanisch realisiert sein können, die Übertragungsverbindung elektrisch mittels Steuer- und Energieversorgungsleitungen zu realisieren.

Das Griffteil 4 ist mit einer Hand und zumindest zwischen Handballen und Mittelhand bzw. den inneren Fingerhandknochen einer Hand umgreifbar. Die Form des Griffteiles 4 ist dabei so an die in Ruhestellung befindliche Innenhand angepaßt, daß das medizinische Handhabungsgerät 1 über einen langen Zeitraum praktisch ermüdungsfrei in der Hand gehalten werden kann.

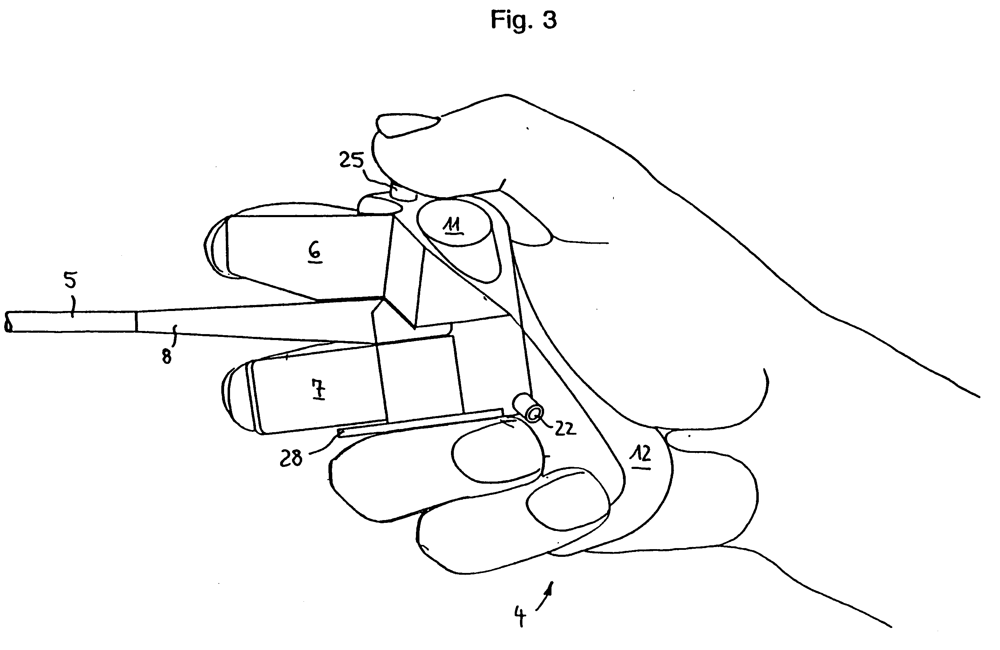

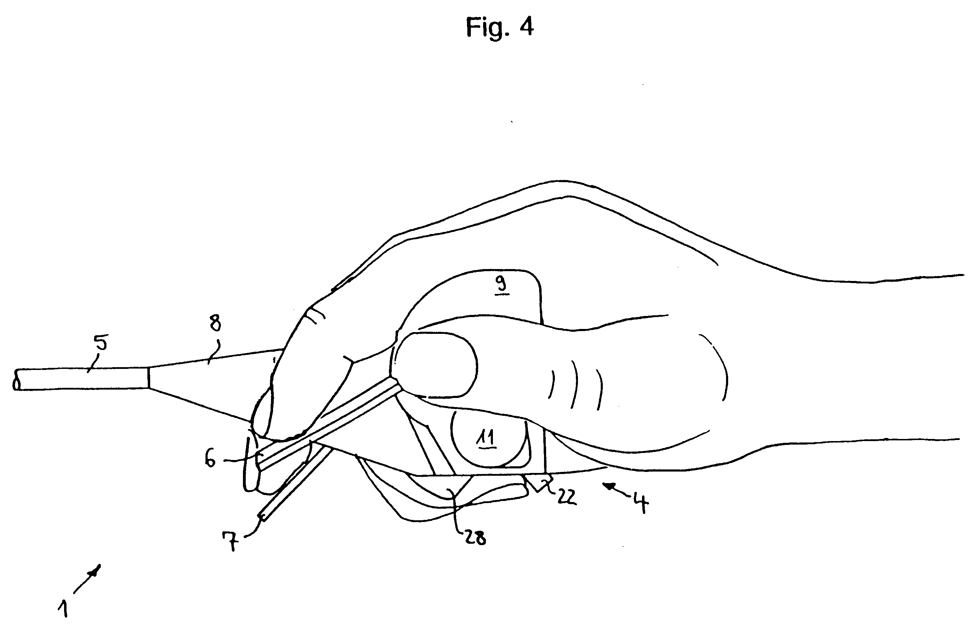

Für den Zeige- und Mittelfinger sind jeweils separate, beispielsweise als Schwenktasten 6, 7 ausgebildete Betätigungselemente vorgesehen, die mit den freien Fingerenden dieser Finger bedienbar sind, wobei die übrigen Hand- und Fingerbereiche im wesentlichen in Greif- oder Haltestellung bleiben können (Figur 3 und 4). Das Handhabungsgerät 1 kann dadurch auch beim Betätigen der Schwenktasten 6 und 7 mit nur einer Hand sicher gehalten werden, so daß bei einer minimal invasiven chirurgischen Operation einerseits die Verletzungsgefahr des Patienten durch eine Fehlpositionierung des medizinischen Operations-Instruments entsprechend vermindert ist, und andererseits der Chirurg nur eine Hand zum Bedienen des Handhabungsgerät benötigt, die andere Hand also für andere Tätigkeiten freibleibt. Besonders vorteilhaft ist auch, daß die separaten, dem Zeige- bzw. dem Mittelfinger zugeordneten Schwenktasten 6,7 unabhängig voneinander und gegebenenfalls gleichzeitig bedient werden können. Selbstverständlich ist es aber auch möglich, anstelle von Schwenktasten andere Betätigungselemente, wie beispielsweise Wippen oder Stellelemente für elektrische Stelleinrichtungen, wie beispielsweise Potentiometer vorzusehen.

Bei der in Fig. 1 dargestellten Ansicht ist gut zu sehen, daß das Schaftteil 5 zwischen der Schwenktaste 6 für den Zeigefinger und der Schwenktaste 7 für den Mittelfinger mit dem Griffteil 4 verbunden. Dabei weist das Schaftteil 5 im Bereich seines proximalen Endes eine Querschnittserweiterung in Richtung der Längsachse der Schwenktasten 6 und 7 bzw. des in Haltestellung befindlichen Zeige- oder Mittelfingers auf, die durch einen Trennsteg 8 gebildet ist. Der Trennsteg 8 ermöglicht einerseits eine gute Biegesteifigkeit des Schaftteiles 5 an der Verbindungsstelle zum Griffteil 4 und bewirkt andererseits eine Seitenführung für den Zeige- und Mittelfinger, die ein Übergreifen der Finger auf die jeweils seitlich benachbarte Schwenktaste verhindert. Außerdem ist das Griffteil 4 in Haltestellung durch den Trennsteg 8 in Querrichtung von Zeige- und Mittelfinger exakt in Bezug zu der Hand positioniert, so daß es jeweils optimal in der Hand liegt und auch bei einem leichten Lösen der Haltestellung praktisch nicht aus der Hand rutschen kann.

Das Schaftteil 5 ist etwa in gerader Verlängerung des gestreckten Hand-Unterarmverlaufes angeordnet (vgl. Figur 4). Wenn das Schaftteil 5 in eine Trokarhülse eingesteckt ist, kann es dadurch bei gestrecktem Hand-Unterarmverlauf auf einfache Weise durch eine Drehung der Hand bzw. des Unterarms in der Trokarhülse um deren Längsachse gedreht werden. Ein seitliches Verschwenken des Armes ist dabei nicht erforderlich.

Im Auflagebereich zwischen Daumen und Handwurzelknochen des Zeigefingers weist das Grifftell 4 einen nach außen vorstehenden Klemmsteg 9 auf, der in der Haltestellung der Hand zwischen Daumen und Handwurzelknochen des Zeigefingers festklemmbar ist. Das Griffteil 4 kann dadurch auch mit dem Daumen gehalten werden, wobei die übrigen Finger aus der Haltestellung gelöst werden können.

Um ein seitliches Abrutschen der Finger an dem Griffteil 4 zu verhindern, sind für den kleinen Finger und den Ringfinger an dem Griffteil 4 in Längsrichtung der in Haltestellung befindlichen Finger verlaufende Einformungen 10 sowie ein Spitzsteg 28 vorgesehen.

Das Griffteil 4 weist ferner eine Daumenauflage 11 mit einer Einformung zum Fixieren des Daumens auf.

Damit das Griffteil 4 bei unterschiedlichen Benutzern noch besser an deren individuelle Hände angepaßt werden kann, ist es mehrteilig ausgebildet und weist ein auswechselbares Ballenteil 12 auf (Figur 5 und 6). Dabei können für ein Handhabungsgerät 1 mehrere, unterschiedlich geformte oder unterschiedlich große, wahlweise mit dem Handhabungsgerät 1 kombinierbare Ballenteile 12 vorgesehen sein. Das Griffteil 4 kann dadurch auf einfache Weise entsprechend den jeweiligen Wünschen des Benutzers gestaltet werden.

Bei dem in Fig. 5 dargestellten Ausführungsbeispiel ist das medizinische Instrument 3 eine Schere. Die Schere ist drehfest mit dem stirnseitigen, distalen Ende einer Hohlwelle 13 verbunden, die das Schaftteil 5 in Längsrichtung durchsetzt und um die Längsachse des Schaftteils 5 drehbar in diesem gelagert ist. An dem proximalen Stirnende der Hohlwelle 13 ist ein Antriebsritzel 14 vorgesehen, daß mit der Verzahnung eines ersten Zwischenzahnrads 15 in Eingriff steht, das etwa den gleichen Durchmesser aufweist wie das Antriebsritzel 14. Das erste Zwischenzahnrad 15 ist einstückig mit einem kleineren, zweiten Zwischenzahnrad 16 verbunden, das mit der Außenverzahnung eines im Daumengriffteil 4 angeordneten Drehelement 17 kämmt. Das medizinische Instrument 3 ist dadurch auf einfache Weise um beliebige Winkel - also auch um mehr als 360 Grad - um die Längsachse des Schaftteils 5 drehbar. Dabei bewirken die Zwischenzahnräder 15 und 16 unter Beibehaltung des Drehsinns eine Übersetzung einer an dem Drehelement 17 aufgebrachten Drehbewegung in eine schnellere Drehbewegung des medizinischen Instruments 3.

Durch die Innenlängshöhlung der Hohlwelle 13 ist eine Zug-/Druckstange 18 geführt, die mit den beiden Scherenteilen 19 des medizinischen Instruments 3 gekoppelt ist. Die Zug-/Druckstange 18 ist in der Hohlwelle 13 axial verschiebbar, wobei die freien Enden der Scherenteile 19 bei einer Schiebebewegung der Zug-/Druckstange 18 in Richtung der Scherenteile 19 voneinander weg und mit einer Schiebebewegung in entgegengesetzter Richtung aufeinander zu bewegt werden. Die Zug-/Druckstange 18 ist gelenkig mit einem Schwenkhebel 20 verbunden, der an einem Endbereich an dem Griffteil 4 schwenbar gelagert und mit seinem anderen Endbereich mit einer Betätigungsstange 21 gelenkig verbunden ist, die ihrerseits schwenkbar an der Schwenktaste 7 gelagert ist. Dabei ist die Lagerstelle etwa mittig an der Unterseite der Schwenktaste 7 angeordnet. An der Schwenktaste 7 ist ferner eine Rückholfeder vorgesehen, so daß die Schere durch Druck auf die Schwenktaste 7 mit dem freien Fingerende des Mittelfingers geschlossen und nach Loslassen der Schwenktaste 7 durch die Federkraft der Rückholfeder wieder in ihre Ausgangsstellung zurückkehrt.

Bei dem in Fig. 5 dargestellten Ausführungsbeispiel sind an dem Griffteil 4 außerdem ein Spülanschluß 22 für eine mit der Schwenktaste 6 betätigbare Spülung sowie ein Sauganschluß 23 vorgesehen, der mit der Drucktaste 24 aktivierbar ist. Durch die Hohlwelle 13 sind dazu entsprechende Saug- und Spülleitungen zu dem medizinischen Instrument 3 geführt.

An der Oberseite des Haltegriffs 2 ist außerdem ein Druckknopf 25 zum Anlegen einer Hochfrequenz an die Scherenteile 19 angeordnet. Dabei kann sowohl eine monopolare Hochfrequenzeinrichtung - mit einer entsprechenden Gegenelektrode an dem zu behandelnden Patienten als auch eine bipolare Hochfrequenzeinrichtung - mit elektrisch gegeneinander isolierten Scherenteilen 19 - verwendet werden.

Wie aus Figur 5 und 6 besonders gut erkennbar ist, können im Bereich des Daumens mehrere, alternativ mit dem Daumen zu bedienende Betätigungselemente vorgesehen sein.

Bei dem Ausführungsbeispiel nach Figur 6 ist der distale Endbereich des Schaftteils 5 in Querrichtung nachgiebig ausgebildet und in der Längsinnen-Höhlung der Hohlwelle - 3 sind vier Bowdenzüge 27 vorgesehen, mit denen der flexible Teilbereich des Schaftteils 5 zur Einstellung des Neigungswinkels des medizinischen Instruments 3 seitlich in beliebige Richtungen ausgerenkt werden kann. Dabei sind jeweils zwei Bowdenzüge 27 paarweise einer gemeinsamen Funktionsebene zugeordnet. Die vier Bowdenzüge 27 sind mit einem Betätigungsknopf 20 gekoppelt, der mit dem Daumen seitlich in beliebige Richtungen verschwenkbar ist. Diese Stellen der einzelnen Bowdenzüge 27 greifen jeweils um 90 Grad in Bezug zur Schwenkachse des Betätigungsknopfes 26 zueinander versetzt und in gleichen Abständen zur Schwenkachse an dem Betätigungsknopf 26 an. Dabei sind jeweils die für eine gemeinsame Funktionsebene vorgesehenen Bowdenzüge 27 paarweise gegenüberliegend an dem Betätigungsknopf 26 angeordnet.

Der Betätigungsknopf 26 ist gegen die Rückstellkraft eines federelastischen Elements eindrückbar und weist eine Rastverriegelung auf, die den Betätigungsknopf 26 ge(en seitliches verschwenken arretiert, wenn dieser nicht manuell beaufschlagt bzw. eingedrückt wird. Der Neigungswinkel des medizinischen Instruments 3 kann also auf einfache Weise verstellt werden, indem der Betätigungsknopf 26 mit dem Daumen eingedrückt und dann seitlich ausgelenkt wird. Nach Loslassen des Betätigungsknopfs 26 rastet dieser automatisch ein, so daß das medizinische Instrument 3, in der zuvor eingestellten Neigungsstellung verbleibt. Der Daumen kann dann gegebenenfalls in seine Ruhestellung zurückkehren. Der Betätigungsknopf 26 ermöglicht eine gleichzeitige Einstellung des Neigungswinkels und der Neigungsrichtung des medizinischen Instruments 3, wobei die Hand in der Haltestellung verbleiben kann. Dabei können gegebenenfalls gleichzeitig mit den Schwenktasten 6 und 7 zusätzliche Funktionen, wie beispielsweise das Durchführen einer Schneidbewegung mit der Schere und/oder das Aktivieren der Spülung oder der Absaugung ausgeführt werden. Das medizinische Instrument 3 kann also - wie dies auch die diversen Doppelpfeile in Figur 5 und 6 zeigen - mit dem Handhabungsgerät 1 in vielfältiger Weise verstellt werden.

Erwähnt werden soll noch, daß das erfindungsgemäße Handhabungsgerät auch für die virtuelle Chirurgie geeignet ist, wenn es mit entsprechenden Sensoren kombiniert wird.

### Gewerbliche Anwendbarkeit

Das vorstehend beschriebene medizinische Handhabungsgerät kann auf allen Gebieten der Medizin und insbesondere in der Endoskopie eingesetzt werden. Besonders vorteilhaft ist seine Anwendung in der minimalinvasiven Chirurgie.

## Patentansprüche

1. Medizinisches Handhabungsgerät (1) insbesondere für endoskopische Anwendungen, mit
- einem distal angeordneten medizinischen Instrument (3),
- einem proximal angeordneten Haltegriff (2), der ein mit der Hand ergreifbares Griffteil (4) aufweist, an dem mit den Fingern bedienbare Betätigungselemente vorgesehen sind, und
- einem Schaftteil (5), das den Haltegriff (2) mit dem Instrument (3) verbindet, und das Übertragungselemente aufweist, die eine Betätigung der Betätigungselemente zu dem medizinischen Instrument (3) übertragen,
**gekennzeichnet** durch die Kombination folgender Merkmale:
- das Griffteil (4) ist derart ergonomisch geformt, daß es weitgehend mit einer Hand zumindest zwischen Handballen und der Mittelhand bzw. den inneren Fingerhandknochen umgriffen und ohne Einsatz zumindest der äußeren und der mittleren Glieder des Zeigefingers und des Mittelfingers gehalten werden kann,
- zumindest für den Zeige- und den Mittelfinger ist jeweils ein separates Betätigungselement vorgesehen, von denen jedes so ausgebildet ist, daß bei seiner Betätigung mit dem freien Fingerende des zugeordneten Fingers die übrigen Hand- und Fingerbereiche im wesentlichen in Greif- bzw. Haltestellung bleiben.

2. Handhabungsgerät nach Anspruch 1,
dadurch **gekennzeichnet**, daß die Betätigungselemente zur proportionalen Übertragung der Betätigungsbewegung ausgebildet sind.

3. Handhabungsgerät nach Anspruch 2,
dadurch **gekennzeichnet**, daß die Betätigungselemente derart ausgebildet sind, daß ihre Stellung die Stellung einer diesem Betätigungselement zugeordneten Schwenk- oder Drehachse für das chirurgische oder medizinische Instrument anzeigt.

4. Handhabungsgerät nach Anspruch 2 oder 3,
dadurch **gekennzeichnet**, daß die Betätigungselemente Analogtaster, Wippen, analoge elektrische Stellelemente und/oder analoge Schwenktasten sind.

5. Handhabungsgerät nach einem der Ansprüche 1 bis 4,
dadurch **gekennzeichnet**, daß die Betätigungselemente gegen eine Rückstellkraft mit dem jeweiligen Fingerende druckbeaufschlagbar sind.

6. Handhabungsgerät nach einem der Ansprüche 1 bis 5,
dadurch **gekennzeichnet**, daß wenigstens ein separates Betätigungselement für den Daumen, insbesondere eine Drucktaste (24), eine Schwenktaste, eine Wippe oder ein Drehelement (17) vorgesehen ist.

7. Handhabungsgerät nach Anspruch 6,
dadurch **gekennzeichnet**, daß im Greifbereich des Daumens mehrere, wahlweise mit dem Daumen betätigbare Elemente angeordnet sind.

8. Handhabungsgerät nach einem der Ansprüche 1 bis 7,
dadurch **gekennzeichnet**, daß das Schaftteil (5) zwischen der Auflage bzw. den Betätigungselementen für den Zeigefinger und den Mittelfinger mit dem Griffteil verbunden ist.

9. Handhabungsgerät nach Anspruch 8,
dadurch **gekennzeichnet**, daß das Schaftteil (5) etwa in gerader Verlängerung des gestreckten Hand-Unterarmverlaufes angeordnet ist.

10. Handhabungsgerät nach einem der Ansprüche 1 bis 9,
dadurch **gekennzeichnet**, daß das Griffteil (4) mehrteilig ausgebildet ist, und
daß insbesondere mehrere, auswechselbare und unterschiedlich große Ballenteile (12) vorgesehen sind.

11. Handhabungsgerät nach einem der Ansprüche 1 bis 10,
dadurch **gekennzeichnet**, daß Betätigungselemente für Öffnungs- und Schließbewegungen sowie gegebenenfalls für eine Rotationsbewegung des medizinischen Instruments (3) vorgesehen sind.

12. Handhabungsgerät nach einem der Ansprüche 1 bis 11,
dadurch **gekennzeichnet**, daß wenigstens ein vorzugsweise durch Betätigungselemente steuerbarer Sauganschluß (23) und/oder Spülanschluß (22) am Handhabungsgerät (1) vorgesehen ist.

13. Handhabungsgerät nach einem der Ansprüche 1 bis 12,
dadurch **gekennzeichnet**, daß zumindest eines der Betätigungselemente mit einer Feststellvorrichtung zum Festlegen des Betätigungselementes in wenigstens einer Endstellung und/oder einer oder mehreren Zwischenstellungen gekoppelt ist.

14. Handhabungsgerät nach einem der Ansprüche 1 bis 13,
dadurch **gekennzeichnet**, daß im Auflagebereich zwischen Daumen und Handwurzelknochen des Zeigefingers ein Klemmsteg (9) vorgesehen ist.

15. Handhabungsgerät nach einem der Ansprüche 1 bis 14,
dadurch **gekennzeichnet**, daß im Bereich der Fingerauflagen für den kleinen Finger und den Ringfinger Einformungen (10) bzw. zwischen und/oder neben den Fingern vorstehende Stützstege (28) vorgesehen sind.

16. Handhabungsgerät nach einem der Ansprüche 1 bis 15,
dadurch **gekennzeichnet**, daß an dem Griffteil (4) eine Daumenauflage (11) vorgesehen ist und daß diese vorzugsweise eine Aufnahmevertiefung oder dergleichen Ausformung für den Daumen aufweist.

17. Handhabungsgerät nach einem der Ansprüche 1 bis 16,
dadurch **gekennzeichnet**, daß das medizinische Instrument (3) um die Längsachse des Schaftteils (5) drehbar an dem Schaftteil (5) gelagert ist,
daß in dem Schaftteil (5) eine Welle vorgesehen ist, die das Schaftteil (5) in Längsrichtung durchsetzt und an ihrem distalen Endbereich drehfest mit dem medizinischen Instrument (3) verbunden ist, und
daß die Welle mit einem an dem Griffteil (4) vorgesehen Betätigungselement in Antriebsverbindung steht.

18. Handhabungsgerät nach einem der Ansprüche 1 bis 17,
dadurch **gekennzeichnet**, daß die Welle direkt oder indirekt über ein Getriebe mit einem vorzugsweise im Daumenbereich an dem Griffteil (4) angeordneten Drehelement oder einem entsprechenden Betätigungselement gekoppelt ist.

19. Handhabungsgerät nach einem der Ansprüche 1 bis 18,
dadurch **gekennzeichnet**, daß die Welle in dem Schaftteil (5) zur Übertragung einer Verstellbewegung des medizinischen Instruments (3), insbesondere einer Öffnungs- und/oder Schließbewegung eines Greifers, einer Schere oder einem anderen Effektor axial verschiebbar geführt ist.

20. Handhabungsgerät nach einem der Ansprüche 1 bis 19,
dadurch **gekennzeichnet**, daß die Welle als Hohlwelle (13) ausgebildet ist, und
daß in deren Innenlängs-Höhlung ein Übertragungselement für Zug- und/oder Druckkräfte und insbesondere eine Zug-/Druckstange (18) oder ein Bowdenzug (27) vorgesehen ist.

21. Handhabungsgerät nach einem der Ansprüche 1 bis 20,
dadurch gekennzeichnet, daß in dem Schaftteil (5) wenigstens eine, gegebenenfalls durch die Innenlängs-Höhlung der Hohlwelle (13) gebildete Leitung zum Zu- oder Abführen einer Flüssigkeit, wie beispielsweise einer Spülflüssigkeit vorgesehen ist.

22. Handhabungserät nach einem der Ansprüche 1 bis 21,
dadurch **gekennzeichnet**, daß an dem Griffteil (4), vorzugsweise in dem Bereich des Daumens ein wenigstens in zwei Richtungen seitlich verschwenkbarer Betätigungsknopf (26) angeordnet ist, und daß an dem Betätigungsknopf (26) wenigstens ein mit dem medizinischen Instrument (3) gekoppeltes Übertragungselement angreift.

23. Handhabungsgerät nach einem der Ansprüche 1 bis 21,
dadurch **gekennzeichnet**, daß der Betätigungsknopf (26) in alle Seitenrichtungen verschwenkbar ist und mehrere, vorzugsweise vier um jeweils 90 Grad versetzt zueinander an dem Betätigungsknopf angreifende Übertragungselemente, vorzugsweise Bowdenzüge (27) aufweist.

24. Handhabungsgerät nach einem der Ansprüche 1 bis 13,
dadurch **gekennzeichnet**, daß das Griffteil zur Reinigung zerlegbar ist und/oder Löcher aufweist bzw. aus Lochstruktur-Elementen besteht.

## Claims

1. A medical manipulating device (1), in particular, for endoscopic applications, having
- a medical instrument (3) disposed at the distal end,
- a grip (2) disposed at the proximal end, said grip (2) being provided with a grasping part (4) which can be held by the hand, said grasping part (4) being provided with actuating elements which can be operated with the fingers, and
- a shank part (5), which connects said grip (2) to said instrument (3), and which is provided with transmission elements which transmit actuation of said actuating elements to said medical instrument (3),
**characterized by** a combination of the following features:
- said grasping part (3) is ergonomically shaped in such a manner that it can be largely grasped by one hand at least between the ball of the hand and the metacarpus respectively the first row of the phalanges of the fingers and can be held without use of at least the third and second phalange of the index finger and the middle finger,
- a separate actuating element is provided for at least the index finger and the middle finger respectively, each said actuating element being designed in such a manner that upon being actuated with the free finger tip of the assigned finger, the rest of the hand and the fingers remain essentially in a grasping respectively holding position.

2. A hand manipulating device according to claim 1,
**characterized by** said actuating elements being designed for proportional transmission of the actuating motion.

3. A hand manipulating device according to claim 2,
**characterized by** said actuating elements being designed in such a manner that their position indicates the position of the swiveling axis or axis of rotation assigned to said actuating element for said surgical or medical instrument.

4. A hand manipulating device according to claim 2 or 3,
**characterized by** said actuating elements being analog tabs, rocker keys, analog electric control elements and/or analog swivel keys.

5. A hand manipulating device according to one of the claims 1 to 4,
**characterized by** said actuating elements being pressable with the respective finger tip against a restoring force.

6. A hand manipulating device according to one of the claims 1 to 5,
**characterized by** at least one separate actuating element being provided for the thumb, in particular, a press button key (24), a swivel key, a rocker key or a rotary element (17).

7. A manipulating device according to claim 6,
**characterized by** multiple elements which can be selectively operated with the thumb being disposed in the grasping range of the thumb.

8. A manipulating device according to one of the claims 1 to 7,
**characterized by** said shank part (5) being connected to said grasping part between the support for respectively the actuating elements for the index finger and the middle finger.

9. A manipulating device according to claim 8,
**characterized by** said shank part (5) being disposed approximately as a straight extension of the outstretched hand-underarm.

10. A manipulating device according to one of the claims 1 to 9,
**characterized by** said grasping part (4) being designed multipartite, and
in particular multiple, exchangeable and different size ball parts (12) being provided.

11. A manipulating device according to one of the claims 1 to 10,
**characterized by** actuating elements for opening and closing motions as well as, if need be, for a rotary motion of said medical instrument (3) being provided.

12. A manipulating device according to one of the claims 1 to 11,
**characterized by** at least one suction connection (23) and/or rinse connection (22), preferably which can be regulated by actuating elements, being provided on the manipulating device (1).

13. A manipulating device according to one of the claims 1 to 12,
**characterized by** at least one of said actuating elements being coupled to a lock-in device for locking said actuating element in at least one final position and/or one or several intermediate positions.

14. A manipulating device according to one of the claims 1 to 13,
**characterized by** a clamping means (9) being disposed in the support region between the thumb and the first row of phalanges of the index finger.

15. A manipulating device according to one of the claims 1 to 14,
**characterized by** molded grooves (10) being provided in the region of the finger supports for the little finger and the ring finger respectively raised separation means (28) between and/or beside the fingers.

16. A manipulating device according to one of the claims 1 to 15,
**characterized by** a thumb support (11) being provided on the grasping part (4) and said thumb support (11) preferably being provided with a receiving recess or a similar molded groove for the thumb.

17. A manipulating device according to one of the claims 1 to 16,
**characterized by** said medical instrument (3) being borne on said shank part (5) in a rotatable manner about the longitudinal axis of said shank part (5), in said shank part (5) a shaft being provided which runs through said shank part (5) in the longitudinal direction and the distal end of said shaft being connected to said medical instrument (3) in a non-rotatable manner, and said shaft being connected to an actuating element provided at said grasping part (4) in a driving manner.

18. A manipulating device according to one of the claims 1 to 17,
**characterized by** said shaft being directly or indirectly coupled via a transmission to a rotary element preferably disposed in the thumb region on the grasping part (4) or to a corresponding actuating element.

19. A manipulating device according to one of the claims 1 to 18,
**characterized by** said shaft being guided in an axially shiftable manner in said shank part (5) to transmit an adjustment motion of said medical instrument (3), in particular, an opening and/or closing motion of a gripper, a pair of scissors or another actuator.

20. A manipulating device according to one of the claims 1 to 19,
**characterized by** said shaft being designed as a hollow shaft (13), and
in the internal longitudinal cavity thereof a transmission element being provided for pull and/or push forces and, in particular, a pull/push rod (18) or a Bowden pull cable (27).

21. A manipulating device according to one of the claims 1 to 20,
**characterized by** in said shank part (5) at least one conduit, if need be formed by said internal longitudinal cavity of said hollow shaft (13), being provided for introducing or removing a fluid, such as by way of illustration a rinsing fluid.

22. A manipulating device according to one of the claims 1 to 21,
**characterized by** an actuating button (26) which can be swiveled laterally in at least two directions being provided on said grasping part (4) preferably in the region of the thumb, and
at least one transmission element coupled with said medical instrument (3) acting on said actuating button (26).

23. A manipulating device according to one of the claims 1 to 21,
**characterized by** said actuating button (26) being swivelable in all directions and multiple, preferably four transmission elements, preferably Bowden pull cables (27) being provided which act 90 degrees off set to each other respectively on said actuating button.

24. A manipulating device according to one of the claims 1 to 21,
**characterized by** said grasping part being disassemblable for cleaning and/or said grasping part being provided with holes respectively being composed of hole-structured elements.

## Revendications

1. Dispositif de maniement (1), en particulier pour des applications en endoscopie, comprenant
- un instrument médical (3) disposé à l'extrémité distale,
- une manche de retenue (2) disposée à l'extrémité proximale et comprenant une partie de manche (4) à être saisie par une main, à laquelle sont disposés des éléments d'actionnement commandable par les doigts, et
- une partie de tige (5) qui relie ladite manche de retenue (2) audit instrument (3) et qui comprend des éléments de transmission pour le transfert d'une commande desdits éléments d'actionnement audit instrument médical (3),
**caractérisé par** la combinaison des caractéristiques suivantes:
- ladite partie de manche (4) présente une forme ergonomique telle qu'elle soit apte à être serrée largement par une seule main au moins entre la paume et et le métacarpe ou respectivement les os des doigts de la main et retenue sans emploi d'au moins des phalanges extérieures et moyennes de l'index et du médius,
- un élément d'actionnement séparé respectif est disposé au moins pour l'index et le médius, dont chacun est configuré de telle façon qu'à un actionnement par l'extrémité libre du doigt affecté les autres zones de la main et des doits reste essentiellement en position de saisie ou respectivement retenue.

2. Dispositif de maniement selon la revendication 1,
**caractérisé en ce** que lesdits éléments d'actionnement sont configurés pour la transmission proportionnelle des mouvements de commande.

3. Dispositif de maniement selon la revendication 2,
**caractérisé en ce** que lesdits éléments d'actionnement sont configurés d'une façon que leur position indique la position d'un axe de pivotement ou de révolution affecté à cet élément d'actionnement pour ledit instrument de chirurgie ou médical.

4. Dispositif de maniement selon la revendication 2 ou 3,
**caractérisé en ce** que lesdits éléments d'actionnement sont des boutons poussoirs analogiques, des boutons à bascule, des éléments de réglage analogiques électrique et/ou des touches orientables analogiques.

5. Dispositif de maniement selon une quelconque des revendications 1 à 4,
**caractérisé en ce** que lesdits éléments d'actionnement sont aptes à être soumis à la pression par l'extrémité du doigt respectif de façon antagoniste à la force rappel.

6. Dispositif de maniement selon une quelconque des revendications 1 à 5,
**caractérisé en ce** qu'au moins un élément d'actionnement séparé est disposé pour le pouce, en particulier sous forme d'une touche (24), d'une touche orientable, d'un bouton à bascule ou d'un élément rotatif (17).

7. Dispositif de maniement selon la revendication 6,
**caractérisé en ce** que dans la zone de saisie du pouce plusieurs éléments sont disposés pour une commande facultative par le pouce.

8. Dispositif de maniement selon une quelconque des revendications 1 à 7,
**caractérisé en ce** que ladite partie de tige (5) est reliée à ladite partie de manche entre l'appui ou respectivement les éléments d'actionnement de l'index et du médius.

9. Dispositif de maniement selon la revendication 8,
**caractérisé en ce** que ladite partie de tige (5) est disposée approximativement de façon à prolonger l'étendue de la main et de l'avant-bras tendus.

10. Dispositif de maniement selon une quelconque des revendications 1 à 9,
**caractérisé en ce** que ladite partie de manche (4) a une configuration à plusieurs parties, et
en ce que plusieurs parties de paume (12) échangeables à tailles différentes sont prévues en particulier.

11. Dispositif de maniement selon une quelconque des revendications 1 à 10,
**caractérisé en ce** que des éléments d'actionnement sont disposés pour les mouvements d'ouverture et de fermeture ainsi qu'éventuellement pour un mouvement rotatoire de l'instrument médical (3).

12. Dispositif de maniement selon une quelconque des revendications 1 à 11,
**caractérisé en ce** qu'au moins un raccord d'aspiration (23) et/ou un raccord de lavage (22) est disposé sur le dispositif de maniement (1), qui est de préférence commandable moyennant des éléments d'actionnement.

13. Dispositif de maniement selon une quelconque des revendications 1 à 12,
**caractérisé en ce** qu'au moins un desdits éléments d'actionnement est accouplé à un dispositif d'arrêt à arrêter l'élément d'actionnement en au moins une position extrême et/ou une ou plusieurs position(s) intermédiaire(s).

14. Dispositif de maniement selon une quelconque des revendications 1 à 13,
**caractérisé en ce** que dans la zone d'appui entre les os de pouce et du carpe de l'index une entretoise de serrage (9) est disposée.

15. Dispositif de maniement selon une quelconque des revendications 1 à 14,
**caractérisé en ce** que des évidements (10) sont formés dans la zone des appuis pour les doigts de l'auriculaire et de l'annulaire ou respectivement des entretoises d'appui (28) sont disposées entre et/ou à côté des doigts.

16. Dispositif de maniement selon une quelconque des revendications 1 à 15,
**caractérisé en ce** qu'un appui de pouce (11) est formé à ladite partie de manche (4), et en ce que cet appui présente, de préférence, un évidement ou une configuration similaire à recevoir le pouce.

17. Dispositif de maniement selon une quelconque des revendications 1 à 16,
**caractérisé en ce** que l'instrument médical (3) est logé à ladite partie de tige (5) de façon à tourner autour l'axe longitudinal de ladite partie de tige (5), en ce qu'un arbre est disposé dans ladite partie de tige (5), qui s'étend à travers ladite partie de tige (5) le long de la direction longitudinale, en étant relié par sa zone d'extrémité distale audit instrument médical (3) de façon non-rotative, et en ce que ledit arbre se trouve en communication d'entraînement avec un élément d'actionnement disposé à ladite partie de manche (4).

18. Dispositif de maniement selon une quelconque des revendications 1 à 17,
**caractérisé en ce** que l'arbre est accouplé directement ou indirectement via une transmission à un élément rotatif ou un élément d'actionnement correspondant, qui est disposé, de préférence, dans la zone de pouce à ladite partie de manche (4).

19. Dispositif de maniement selon une quelconque des revendications 1 à 18,
**caractérisé en ce** que ledit arbre dans la partie de tige (5) est guidé pour un mouvement de déplacement axial pour la transmission d'un mouvement d'ajustage dudit instrument médical (3), en particulier d'un mouvement d'ouverture et/ou de fermeture d'un élément preneur, d'une paire de ciseaux ou d'un autre élément d'effet.

20. Dispositif de maniement selon une quelconque des revendications 1 à 19,
**caractérisé en ce** que ledit arbre a la configuration d'un arbre creux (13), et en ce qu'un élément de transmission à transférer des efforts de traction et/ou des forces de compression, et en particulier une tige de traction/compression (18) ou un élément de transmission de Bowden (27), est disposé dans le creux longitudinal intérieur dudit arbre creux.

21. Dispositif de maniement selon une quelconque des revendications 1 à 20,
**caractérisé en ce** qu'au moins une conduite d'alimentation ou de décharge d'un liquide, par exemple un liquide de lavage, est disposée dans ladite partie de tige (5), qui est éventuellement formée par ledit creux longitudinal intérieur dudit arbre creux (13).

22. Dispositif de maniement selon une quelconque des revendications 1 à 21,
**caractérisé en ce** qu'un bouton de commande (26) est disposé à ladite partie de manche, de préférence dans la zone de pouce, qui est latéralement pivotable en au moins deux directions, et en ce qu'au moins un élément de transmission s'engage audit bouton de commande (26), qui est accouplé audit instrument médical (3).

23. Dispositif de maniement selon une quelconque des revendications 1 à 21,
**caractérisé en ce** que ledit bouton de commande (26) est pivotable en tous les directions latérales et comprend une pluralité, de préférence en nombre de quatre, d'éléments de transmission, de préférence des systèmes de transmission de Bowden (27), qui sont arrangés à un déport mutuel par 90 degrés et qui s'engagent audit bouton de commande.

24. Dispositif de maniement selon une quelconque des revendications 1 à 13,
**caractérisé en ce** que ladite partie de manche est démontable pour le nettoyage et/ou présente des trous ou respectivement est constitué par des éléments à structure perforée.
